# EUROPÄISCHE PATENTSCHRIFT

(11) **EP 0 596 246 B1**
(45) Veröffentlichungstag und Bekanntmachung des Hinweises auf die Patenterteilung: **28.06.1995**
(21) Anmeldenummer: 93115671.5
(22) Anmeldetag: 29.09.1993
(51) Int. Cl.: C07C 50/16, C07C 33/042, C07C 33/03, C07C 11/12

(54) **Neue 2-tert.-Amylverbindungen**
2-tert-amyl compounds
Composés 2-tert.-amyliques

(30) Priorität: 05.10.1992 DE 4233387
(43) Veröffentlichungstag der Anmeldung: 11.05.1994
(73) Patentinhaber: BASF Aktiengesellschaft, 67063 Ludwigshafen (DE)
(72) Erfinder: Ebel, Klaus, Dr., D-6700 Ludwigshafen (DE); Schroeder, Juergen, Dr., D-6806 Viernheim (DE)

(56) Entgegenhaltungen:
- EP-A- 0 044 413
- DE-A- 2 460 922
- FR-A- 946 980

## Beschreibung

Die vorliegende Erfindung betrifft neue 2-tert.-Amylverbindungen, Verfahren zu deren Herstellung sowie die Umsetzung von 2-tert.-Amylbutadien zu 2-tert.-Amylanthrachinon [2-(2-Methylbut-2-yl)-anthrachinon].

Aus Chem. Abstr., Vol. 80, 47 709 (1974) ist die Herstellung von 2-tert.-Amylanthrachinon durch Umsetzung von tert.-Amylbenzol mit dem fünffachen Überschuß an Phthalsäureanhydrid bekannt, um die Isomerisierung der tert.-Amylgruppe zurückzudrängen und die anschließende Cyclisierung der entstandenen (4-tert.-Amylbenzoyl)-benzoesäure mit konz. Schwefelsäure beschrieben.

Aus der DE-A-27 20 294 ist ein Verfahren zur Herstellung von 2-tert.-Amylanthrachinon durch Friedel-Crafts-Acylierung bekannt, bei dem der freiwerdende Chlorwasserstoff mit Luft herausgestrippt werden muß, um die Isomerisierung der tert.-Amylgruppe zurückzudrängen.

Aus der EP-A-248 423 ist bekannt, daß die Isomerisierung der tert.-Amylgruppe bei der Friedel-Crafts-Acylierung von Phthalsäureanhydrid mit tert.-Amylbenzol zurückgedrängt wird, wenn der freiwerdende Chlorwasserstoff durch Reaktion mit einem Alkylaluminiumchlorid abgefangen wird.

In DE-A-20 13 299 ist ein Verfahren zur Herstellung von tert.-Amylanthrachinon beschrieben, bei dem (4-tert.-Amylbenzoyl)-benzoesäure in einer Grignard-Reaktion von Phthalsäureanhydrid mit 4-tert.-Amylphenylmagnesiumchlorid hergestellt wird.

Die bisherigen Verfahren haben den Nachteil, daß bei der Herstellung von 2-tert.-Amylanthrachinon große Salzmengen anfallen und die Isomerisierung der tert.-Amylgruppe durch zusätzliche Maßnahmen unterdrückt werden muß.

Der vorliegenden Erfindung lag daher die Aufgabe zugrunde, den zuvor genannten Nachteilen abzuhelfen.

Demgemäß wurden neue tert.-Amylverbindungen der allgemeinen Formel I 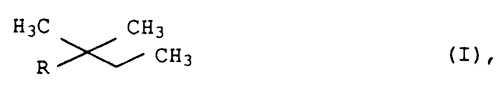
in der 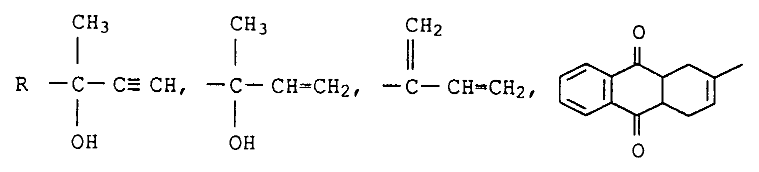
bedeuten, gefunden, sowie neue Verfahren zur Herstellung von 2-tert.- Amylbutadien durch Dehydratisierung von 3,4,4-Trimethylhex-1-en-3-ol bei Temperaturen von 100 bis 350°C und Drücken von 0,01 bis 50 bar an sauren Katalysatoren, deren Herstellung durch Partialhydrierung von 3,4,4-Trimethylhex-1-in-3-ol bei Temperaturen von 0 bis 50°C und Drücken von 0,01 bis 50 bar und deren Herstellung durch Umsetzung von tert.-Amylmethylketon (3,3-Dimethylpentan-2-on) mit Acetylen in Gegenwart von basischen Katalysatoren bei Temperaturen von 0 bis 60°C und Drücken von 0,01 bis 50 bar, ferner die Umsetzung von 2-tert.- Amylbutadien mit 1,4-Naphthochinon bei Temperaturen von 20 bis 200°C und Drücken von 0,01 bis 50 bar zu 2-tert.-Amyl-1,4,4a,9a-tetrahydroanthrachinon und anschließende Oxidation in Gegenwart einer starken Base bei Temperaturen von 0 bis 50°C.

Es wurden die folgenden neuen Verbindungen gefunden:
- 2-tert.-Amylbutadien
- 3,4,4-Trimethylhex-1-en-3-ol
- 3,4,4-Trimethylhex-1-in-3-ol
- 2-tert.-Amyl-1,4,4a,9a-tetrahydroanthrachinon = 2-(2-Methylbut-2-yl)-1,4,4a,9a-tetrahydroanthrachinon

Die erfindungsgemäßen Verfahren lassen sich wie folgt durchführen:
- Herstellung von 2-tert.-Amylbutadien

3,4,4-Trimethylhex-1-en-3-ol kann bei Temperaturen von 100 bis 350°C und Drücken von 0,01 bis 50 bar in der Flüssigphase oder bevorzugt in der Gasphase an sauren Katalysatoren dehydratisiert werden, z.B. auch nach Houben-Weyl, Methoden der organischen Chemie, Band 5/1b, Seite 45 bis 104. Das entstehende 2-tert.-Amylbutadien kann bevorzugt kontinuierlich abdestilliert werden und als Rohprodukt direkt weiterverarbeitet oder nochmals destilliert werden.

Für die Flüssigphasenfahrweise eignen sich Temperaturen von 100 bis 300°C, bevorzugt 130 bis 250°C, besonders bevorzugt 140 bis 200°C. In der Regel legt man ein inertes hochsiedendes Lösungsmittel und den sauren Katalysator vor und tropft das 3,4,4-Trimethylhex-1-en-3-ol zu.

Als saure Katalysatoren eignen sich in der Regel alle sauren Heterogen-, bevorzugt Homogenkatalysatoren. Als saure Homogenkatalysatoren seien beispielsweise Mineralsäuren wie Schwefelsäure und Phosphorsäure genannt.

Die sauren Homogenkatalysatoren werden in der Regel im Molverhältnis von 0,001 : 1 bis 0,05 : 1, bevorzugt 0,005 : 1 bis 0,04 : 1, besonders bevorzugt 0,01 : 1 bis 0,02 : 1 zum 3,4,4-Trimethylhex-1-en-3-ol eingesetzt.

Als inerte hochsiedende Lösungsmittel eignen sich beispielsweise-Polyethylen-glykol, Polypropylenglykol, Vakuumgasöl und technisches Weißöl, bevorzugt Polyethylenglykol.

In der Gasphasenfahrweise werden Lösungsmittel in der Regel nicht benötigt. Die Reaktionstemperaturen liegen bei 150 bis 350°C, bevorzugt bei 180 bis 300°C, besonders bevorzugt bei 200 bis 250°C. Das den Reaktor verlassende Gasgemisch wird in der Regel kondensiert. Die organische Phase kann destilliert oder direkt weiterverarbeitet werden.

Als saure Katalysatoren eignen sich in der Regel alle sauren Homogen-, bevorzugt Heterogenkatalysatoren. Als saure Heterogenkatalysatoren seien beispielsweise sauer wirkende Oxide von Elementen der III. und IV. Hauptgruppe sowie der IV. bis VI. Nebengruppe des Periodensystems der Elemente genannt, wie Siliciumdioxid in Form von Kieselgel, Kieselgur, Quarz, Titandioxid, Zirkondioxid, Phosphorpentoxid, Vanadiumpentoxid, Bortrioxid, Aluminiumoxid, Chromoxide, Molybdänoxide, Wolframoxide oder deren Gemische gegebenenfalls mit einem Zusatz von Phosphorsäure, zweckmäßig in einer Menge von 1 bis 30 Gew.-%, insbesondere 5 bis 15 Gew.-% Phosphorsäure genannt, bevorzugt gamma-Aluminiumoxid.
- Herstellung von 3,4,4-Trimethylhex-1-en-3-ol

3,4,4-Trimethylhex-1-in-3-ol kann bei Temperaturen von 0 bis 50°C, bevorzugt 5 bis 20°C und Drücken von 0,01 bis 50 bar, bevorzugt 0,1 bis 10 bar, besonders bevorzugt 0,5 bis 5 bar in einer Wasserstoffatmosphäre, gegebenenfalls in einem inerten Lösungsmittel vorgelegt an Partialhydrierkatalysatoren hydriert werden wie z.B. nach Houben- Weyl, Methoden der organischen Chemie, Band 4/1c, Seite 107 bis 109. Nach beendeter Wasserstoffaufnahme wird der Katalysator in der Regel abfiltriert und das Lösungsmittel abdestilliert. Das Rohprodukt kann direkt weiterverarbeitet oder nochmals destilliert werden.

Als Partialhydrierkatalysatoren eignen sich beispielsweise Lindlar-Katalysatoren, die z. B. mit Chinolin vergiftet sind.

Das Gewichtsverhältnis von 3,4,4-Trimethylhex-1-in-3-ol zum Partialhydrierkatalysator beträgt in der Regel 1 : 1 bis 1000 : 1, bevorzugt 10 : 1 bis 500 : 1, besonders bevorzugt 50 : 1 bis 300 : 1.

Als inerte Lösungsmittel eignen sich beispielsweise C₄- bis C₃₀-Alkane wie Butane, Pentane, Hexane, Heptane, Octane und Petrolether, bevorzugt Petrolether.
- Herstellung von 3,4,4-Trimethylhex-1-in-3-ol

2-tert.-Amylmethylketon kann bei Temperaturen von 0 bis 60°C, bevorzugt 10 bis 40°C, besonders bevorzugt 15 bis 35°C und Drücken von 0,01 bis 50 bar, bevorzugt 1 bis 30 bar, besonders bevorzugt 5 bis 25 bar, gegebenenfalls in Gegenwart eines inerten Lösungsmittels, mit Acetylen in Gegenwart von basischen Katalysatoren umgesetzt werden, z.B. auch nach Annalen der Chemie, Band 596, 1 bis 224 (...). Die Aufarbeitung kann nach allgemein bekannten Methoden durchgeführt werden wie z.B. durch Neutralisation mit Ameisensäure, Abfiltrieren und gegebenenfalls Destillieren des Rohproduktes.

Als basische Katalysatoren eignen sich beispielsweise Alkali- und Erdalkalimetallalkoholate. Als Alkalimetallalkoholate eignen sich Lithium-, Natrium-, Kalium-, Rubidium- und Cäsiumalkoholate, bevorzugt Lithium-, Natrium- und Kaliumalkoholate beliebiger Alkohole wie C₁- bis C₂₀-Alkohole. In der Regel verwendet man Alkalimetallalkoholate von C₁- bis C₄-Alkoholen wie Natriummethylat, Natriumethylat, Kaliummethylat, Kaliumethylat und Kalium-tert.-butylat, bevorzugt Alkalimetallalkoholate von C₁- und C₂-Alkoholen wie Natriummethylat, Natriumethylat, Kaliummethylat und Kaliumethylat, besonders bevorzugt Natriummethylat.

Das Molverhältnis von basischem Katalysator (z.B. Alkalimetallalkoholat) zum tert.-Amylmethylketon beträgt in der Regel 0,001 : 1 bis 1 : 1, bevorzugt 0,01 : 1 bis 0,5 : 1, besonders bevorzugt 0,02 : 1 bis 0,2 : 1.

Die basischen Katalysatoren wie die Alkalimetallalkoholate können als Feststoffe in die Reaktion eingesetzt werden. Aus verfahrenstechnischen Gründen ist es jedoch vorteilhaft, mit Lösungen der Katalysatoren zu arbeiten.

Verwendung finden können im Prinzip alle Lösungsmittel, die gegen die verwendeten basischen Katalysatoren und unter den Reaktionsbedingungen stabil sind wie alle gängigen Alkohole wie C₁- bis C₂₀-Alkohole, bevorzugt C₁- bis C₄-Alkohole wie Methanol, Ethanol, Propanol, iso- Propanol, Butanol, sec. -Butanol, iso-Butanol und tert-Butanol, besonders bevorzugt Methanol und Ethanol, acyclische und cyclische Ether wie Diethylether, Methyl-tert.-butylether, Tetrahydrofuran und Dioxan, bevorzugt Tetrahydrofuran.

Als besonders vorteilhaft erweist sich eine alkoholische Lösung der Alkoholate, da diese Lösungen einfach hergestellt werden können, z.B. durch Auflösen der jeweiligen Metalle in dem betreffenden Alkohol. Besonders bevorzugt ist eine Lösung von Natriummethylat in Methanol.

Die Lösungsmittelmenge kann in weiten Bereichen gewählt werden, sie liegt jedoch zweckmäßigerweise bei 50 bis 500 ml, bevorzugt bei 70 bis 200 ml Alkohol pro Mol 2-tert.-Amylmethylketon.

Ferner kann 2-tert.-Amylmethylketon bei Temperaturen von (-20) bis 50°C, bevorzugt (-10) bis 30°C, besonders bevorzugt 0 bis 20°C und Drücken von 0,01 bis 50 bar, bevorzugt 0,1 bis 5 bar, besonders bevorzugt bei Normaldruck (Atmosphärendruck), gegebenenfalls in Gegenwart eines inerten Lösungsmittels, mit Acetylengrignardlösungen zu den entsprechenden Propargylalkoholen umsetzen werden, z.B. analog Preparative Acetylenic Chemistry, 2. Aufl., Verlag ELSEVIER.

Als inerte Lösungsmittel eignen sich beispielsweise acyclische und cyclische Ether wie Diethylether, Methyl-tert.-butylether, Tetrahydrofuran und Dioxan, bevorzugt Tetrahydrofuran.

Das Molverhältnis von der Acetylengrignardverbindung zum tert.-Amylmethylketon beträgt in der Regel 0,7 : 1 bis 3 : 1, bevorzugt 0,9 : 1 bis 2 : 1, besonders bevorzugt 1 : 1 bis 1,5 : 1. Die Hydrolyse kann nach allgemein bekannten Bedingungen durchgeführt werden, z.B. mit 2N Salzsäure, anschließender Abtrennung der organischen Phase und gegebenenfalls Destillation des Produktes.
- Umsetzung von 2-tert.-Amylbutadien

2-tert.-Amylmethylketon und 1,4-Naphthochinon können durch Diels-Alder-Reaktion bei Temperaturen von 20 bis 200°C, bevorzugt 50 bis 120°C und Drücken von 0,01 bis 50 bar, bevorzugt 0,1 bis 5 bar, besonders bevorzugt Normaldruck (Atmosphärendruck) in Gegenwart eines inerten Lösungsmittels zu 2-tert.-Amyl-1,4,4a,9a-tetrahydroanthrachinon umgesetzt werden und anschließend, gegebenenfalls nach destillativer Rückführung nicht umgesetzten 2-tert.-Amylbutadiens, in Gegenwart einer starken Base mit Luft bei Temperaturen von 0 bis 50°C, bevorzugt 15 bis 40°C, oxidiert werden, z.B. analog Org. Synth., Coll. Vol. III, 310 bis 311 (1955).

Das Molverhältnis von 1,4-Naphthochinon zum 2-tert.-Amylbutadien liegt im allgemeinen bei 0,2 : 1 bis 5 : 1, bevorzugt 0,5 : 1 bis 2 : 1, besonders bevorzugt 1 : 1 bis 1,5 : 1.

Als inerte Lösungsmittel eignen sich beispielsweise C₁- bis C₂₀-Alkohole, bevorzugt C₁- bis C₄-Alkohole wie Methanol, Ethanol, Propanol, iso-Propanol, Butanol, sec.-Butanol, iso-Butanol und tert.-Butanol.

Als starke Basen eignen sich beispielsweise Hydroxide wie Lithiumhydroxid, Natriumhydroxid und Kaliumhydroxid, bevorzugt Kaliumhydroxid.

Die erfindungsgemäßen Verbindungen 2-tert.-Amylbutadien, 3,4,4-Trimethylhex-1-en-3-ol, 3,4,4-Trimethylhex-1-in-3-ol, und 2-tert.-Amyl-1,4,4a,9a-tetrahydroanthrachinon [2-(2-Methyl-but-2-yl)-1,4,4a,9a-tetrahydroanthrachinon] eignen sich zur Herstellung von 2-tert.-Amylanthrachinon. 2-tert.-Amylanthrachinon wird als Katalysator für die H₂O₂-Synthese eingesetzt (Ullmanns Encyclopädie der technischen Chemie, 4. Auflage, Band 17, Seite 694 bis 711).

### Beispiele

- Herstellung von 3,4,4-Trimethylhex-1-in-3-ol

### Beispiel 1

32 g tert.-Amylmethylketon und 15 g Natriummethanolat wurden in einem Autoklaven in 50 ml Tetrahydrofuran suspendiert und Acetylen bei Raumtemperatur unter Rühren bis zu einem konstanten Druck von 20 bar aufgepresst. Nach beendeter Reaktion wurde entspannt, mit Ameisensäure neutralisiert, filtriert und das Lösungsmittel abdestilliert. Der Umsatz betrug 66 % bei einer Selektivität von 90 %.

### Beispiel 2

50 g tert.-Amylmethylketon wurden in 50 ml Tetrahydrofuran gelöst und bei 0°C zu 330 ml einer 1,5 molaren Acetylengrignardlösung in Tetrahydrofuran getropft. Nach 30 min. wurde mit 2N Salzsäure hydrolysiert, die organische Phase abgetrennt, das Lösungsmittel entfernt und anschließend fraktionierend destilliert. Man erhielt 52 g (85 %) 3,4,4-Trimethylhex-1-in-3-ol (Sdp.: 110°C/175 mbar).
- Herstellung von 3,4,4-Trimethylhex-1-en-3-ol

### Beispiel 3

100 g 3,4,4-Trimethylhex-1-in-3-ol, 3 g Chinolin und 5,4 g Lindlar-Katalysator wurden in 100 ml Petrolether vorgelegt und bei 25°C 10 Stunden unter Wasserstoffatmosphäre gerührt. Anschließend wurde vom Katalysator abfiltriert, das Filtrat eingeengt und der Rückstand mittels GC analysiert. Man erhielt 91 g (90 %) 3,4,4-Trimethylhex-1-en-3-ol. Der Rückstand wurde ohne Reinigung weiterverarbeitet.

Zur Charakterisierung wurde ein Teil des Produkts destilliert. Der Siedepunkt betrug 78°C bei 30 mbar.
- Herstellung von 2-tert.-Amylbutadien

### Beispiel 4

40 g 3,4,4-Trimethylhex-1-en-3-ol wurden innerhalb von zwei Stunden bei 285°C bis 290°C in ein Gemisch aus 200 g Polyethylenglykol und 3 g Phosphorsäure getropft. Vom zweiphasigen Austrag wurde die organische Phase mittels GC analysiert. Man erhielt 24 g (70 %) 2-tert.-Amylbutadien. Die organische Phase wurde ohne Reinigung weiterverarbeitet.

### Beispiel 5

Über einen Verdampfer wurden stündlich 25 g 3,4,4-Trimethylhex-1-en-3-ol verdampft und bei 220°C über 100 ml eines Katalysators aus gamma-Aluminiumoxid geleitet. Anschließend wurden die Reaktionsgase abgekühlt und in einer Vorlage gesammelt. Anschließend wurden die Phasen getrennt und die organische Phase mittels GC analysiert. Man erhielt 35 g (80 %) 2-tert.-Amylbutadien.

Zur Charakterisierung wurde ein Teil des Produkts destilliert. Der Siedepunkt betrug 115 bis 120°C bei 700 mbar.
- Herstellung von 2-tert.-Amylanthrachinon

### Beispiel 6

20 g 2-tert.-Amylbutadien, 25 g 1,4-Naphthochinon und 300 ml Butanol wurden 8 Stunden auf 70°C erhitzt. Anschliessend wurde mit 30 g Kaliumhydroxid versetzt und 8 Stunden bei 25°C bis 33°C Luft eingeleitet. Nach Entfernung des Lösungsmittel und Umkristallisation aus Cyclohexan erhielt man 35 g (80 %) 2-tert.Amylanthrachinon vom Schmelzpunkt 75 bis 76°C.

## Patentansprüche

1. Tert.-Amylverbindungen der allgemeinen Formel I 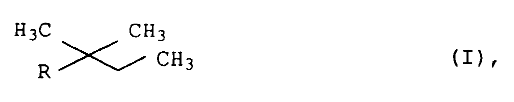 in der 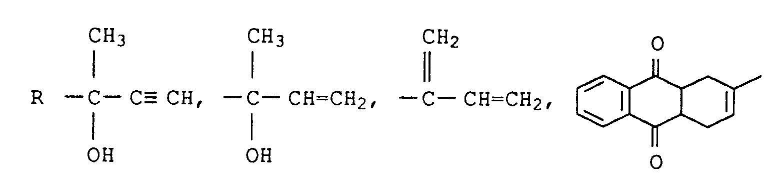 bedeuten.

2. Verfahren zur Herstellung von 2-tert.-Amylbutadien, dadurch gekennzeichnet, daß man 3,4,4-Trimethylhex-1-en-3-ol bei Temperaturen von 100 bis 350°C und Drücken von 0,01 bis 50 bar an sauren Katalysatoren dehydratisiert.

3. Verfahren zur Herstellung von 2-tert.-Amylbutadien nach Anspruch 2, dadurch gekennzeichnet, daß man 3,4,4-Trimethylhex-1-en-3-ol durch Umsetzung von 3,4,4-Trimethylhex-1-in-3-ol an Partialhydierkatalysatoren bei Temperaturen von 0 bis 50°C und Drücken von 0,01 bis 50 bar erhält.

4. Verfahren zur Herstellung von 2-tert.-Amylbutadien nach Anspruch 3, dadurch gekennzeichnet, daß man 3,4,4-Trimethylhex-1-in-3-ol durch Umsetzung von tert.-Amylmethylketon (3,3-Dimethylpentan-2-on) mit Acetylen in Gegenwart von basischen Katalysatoren bei Temperaturen von 0 bis 60°C und Drücken von 0,01 bis 50 bar erhält.

5. Verfahren zur Umsetzung von 2-tert.-Amylbutadien nach Anspruch 2, dadurch gekennzeichnet, daß man 2-tert.-Amylbutadien und 1,4-Naphthochinon bei Temperaturen von 20 bis 200°C und Drücken von 0,01 bis 50 bar zu 2-tert.-Amyl-1,4,4a,9a-tetrahydroanthrachinon umsetzt und anschließend in Gegenwart einer starken Base bei Temperaturen von 0 bis 50°C oxidiert.

## Claims

1. A tert-amyl compound of the general formula I 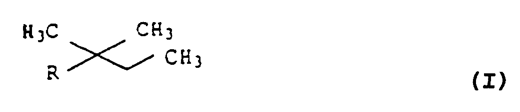 where R is

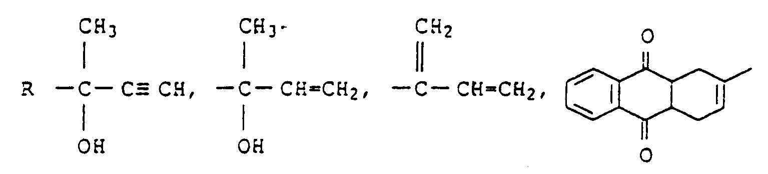

2. A process for preparing 2-tert-amylbutadiene, which comprises dehydrating 3,4,4-trimethylhex-1-en-3-ol at from 100 to 350°C and from 0.01 to 50 bar on acidic catalysts.

3. A process for preparing 2-tert-amylbutadiene as claimed in claim 2, wherein 3,4,4-trimethylhex-1-en-3-ol is obtained by reacting 3,4,4-trimethylhex-1-yn-3-ol on partial hydrogenation catalysts at from 0 to 50°C and from 0.01 to 50 bar.

4. A process for preparing 2-tert-amylbutadiene as claimed in claim 3, wherein 3,4,4-trimethylhex-1-yn-3-ol is obtained by reacting tert-amyl methyl ketone (3,3-dimethylpentan-2-one) with acetylene in the presence of basic catalysts at from 0 to 60°C and from 0.01 to 50 bar.

5. A process for preparing 2-tert-amylanthraquinone, which comprises reacting 2-tert-amylbutadiene and 1,4-naphthoquinone at from 20 to 200°C and from 0.01 to 50 bar to give 2-tert-amyl-1,4,4a,9a-tetrahydroanthraquinone and then oxidizing the latter at from 0 to 50°C in the presence of a strong base.

## Revendications

1. Composés tert-amyliques de la formule générale I 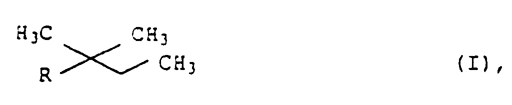 dans laquelle R représente les radicaux qui suivent

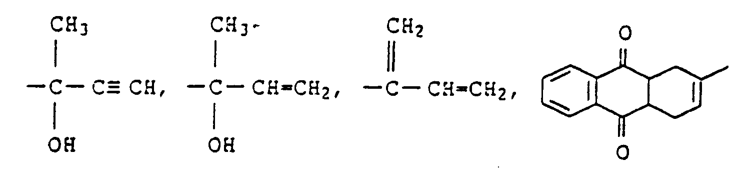

2. Procédé de préparation du 2-tert-amylbutadiène, caractérisé en ce que l'on déshydrate le 3,4,4-triméthylhex-1-ène-3-ol à des températures de 100 à 350°C et sous des pressions de 0,01 à 50 bars, sur des catalyseurs acides.

3. Procédé de préparation du 2-tert-amylbutadiène suivant la revendication 2, caractérisé en ce que l'on obtient le 3,4,4-triméthylhex-1-ène-3-ol par la réaction du 3,4,4-triméthylhex-1-yne-3-ol sur des catalyseurs d'hydrogénation partielle à des température de 0 à 50°C et sous des pressions de 0,01 à 50 bars.

4. Procédé de préparation du 2-tert-amylbutadiène suivant la revendication 3, caractérisé en ce que l'on obtient le 3,4,4-triméthylhex-1-yne-3-ol par la réaction de la tert-amylméthylcétone (3,3-diméthylpentane-2-one) avec l'acétylène en présence de catalyseurs basiques, à des températures de 0 à 60°C et sous des pressions de à,01 à 50 bars.

5. Procédé de préparation du 2-tert-amylbutadiène suivant la revendication 2, caractérisé en ce que l'on fait réagir le 2-tert-amylbutadiène et la 1,4-naphtoquinone à des températures de 20 à 200°C et sous des pressions de 0,01 à 50 bars de manière à obtenir la 2-tert-amyl-1,4,4a,9a-tétrahydroanthraquinone et on procède ensuite à l'oxydation à des températures de 0 à 50°C en présence d'une base forte.
